Europäisches Patentamt

European Patent Office

Office européen des brevets

(11) Publication number: **0 012 376**
**B1**

(19)

(12) **EUROPEAN PATENT SPECIFICATION**

(45) Date of publication of patent specification: **21.04.82**

(21) Application number: **79104967.9**

(22) Date of filing: **06.12.79**

(51) Int. Cl.³: **C 07 D 307/08,**
**C 07 C 31/20, C 07 C 27/02**

(54) Process for producing tetrahydrofuran and 1,4-butanediol.

(30) Priority: **12.12.78 JP 152724/78**

(43) Date of publication of application:
**25.06.80 Bulletin 80/13**

(45) Publication of the grant of the patent:
**21.04.82 Bulletin 82/16**

(84) Designated Contracting States:
**DE GB**

(56) References cited:
**DE - A - 2 611 423**

**PATENTS ABSTRACTS OF JAPAN, vol. 2, no.**
**122, 13 October 1978 pages 2341 C 78**

(73) Proprietor: **JAPAN SYNTHETIC RUBBER CO., LTD.**
**11-24, Tsukiji-2-chome Chuo-ku**
**Tokyo (JP)**

(72) Inventor: **Yoshida, Yoshinori**
**29, Aobadai-2-chome Midori-ku**
**Yokohama (JP)**
Inventor: **Oka, Hiroshi**
**5-17, Hamadayama-4-chome Suginami-ku**
**Tokyo (JP)**

(74) Representative: **Patentanwälte Beetz sen. - Beetz**
**jr. Timpe - Siegfried - Schmitt-Fumian**
**Steinsdorfstrasse 10**
**D-8000 München 22 (DE)**

# 0 012 376

### Process for producing tetrahydrofuran and 1,4-butanediol

This invention relates to a process for producing simultaneously tetrahydrofuran and 1,4-butanediol in any desired proportion from 1,4-diacetoxybutane.

Tetrahydrofuran and 1,4-butanediol are both quite useful as organic solvents or as starting materials for the production of high polymers; they are manufactured by various processes. For example, it is known that tetrahydrofuran can be produced by hydrogenation of furan obtained by decarbonylating furfural, by dehydrating cyclization of 1,4-butanediol obtainable by hydrogenating either butynediol, which can be produced from acetylene and formaldehyde, or 2-butene-1,4-diol which can be obtained from 1,4-dichlorobutene, by hydrogenation of maleic anhydride or by reacting 1,4-diacetoxybutane with water in the presence of an acidic catalyst. It is also known that 1,4-butanediol can be produced by hydrogenation of butynediol, by hydrolysis or transesterification of 1,4-diacetoxybutane.

As to the simultaneous production of tetrahydrofuran and 1,4-butanediol, a process for producing tetrahydrofuran in any desired proportion by dehydrating cyclization of a part of the 1,4-butanediol obtained by various methods and a process for producing tetrahydrofuran and 1,4-butanediol in any desired proportion by controlling the amount of water used, the reaction temperature and the residence time in hydrolyzing acetic esters of 1,4-butanediol, particularly, have been disclosed hitherto. However, in the simultaneous production process in which tetrahydrofuran is produced via 1,4-butanediol there occurs the economical disadvantage that the costs of tetrahydrofuran become higher than the value obtainable by multiplying the costs of 1,4-butanediol by the inverse ratio of molecular weight. In the process by which tetrahydrofuran and 1,4-butanediol are simultaneously produced by hydrolyzing 1,4-diacetoxybutane, there is the drawback that (as mentioned e.g. in the Japanese Patent Application Kokai (Laid-Open) No. 87,305/78) the equilibrium constant of the hydrolysis reaction regarding 1,4-butanediol is so small that the amount of water used becomes very large which requires a great thermal load in the subsequent separation of water by distillation.

The present inventors have conducted extensive research on the process for producing simultaneously tetrahydrofuran and 1,4-butanediol with an industrial advantage from 1,4-diacetoxybutane which is the diacetic ester of 1,4-butanediol. As a result, it has been found, entirely different from the aforementioned case of the Japanese Patent Application Kokai (Laid-Open) No. 87,305/78, that tetrahydrofuran can be obtained with a greatly enhanced conversion of water if the cyclization reaction is carried out by using water in an amount not larger than the theoretical quantity based on 1,4-diacetoxybutane, that 1,4-butanediol can be obtained in a quantitative yield by subjecting the residual liquid (mainly comprising the unreacted acetic ester) obtained by separating the tetrahydrofuran from the reaction mixture to subsequent transesterification with methanol, that the amount of methanol as well as the amount of catalyst used in this case can be made smaller than those used in the direct transesterification of 1,4-diacetoxybutane because the residual liquid comprising mainly the unreacted acetic ester, to be subjected to the transesterification, contains 1-hydroxy-4-acetoxybutane formed by partial hydrolysis and 1,4-butanediol formed by complete hydrolysis and that tetrahydrofuran and 1,4-butanediol can be produced in any desired proportion by selecting the amount of unreacted acetic ester to be subjected to the transesterification depending on the desired yield of 1,4-butanediol and recycling the residual portion to the cyclization reaction.

Accordingly, the purpose of the invention is to provide a process for producing tetrahydrofuran and 1,4-butanediol simultaneously in any desired proportion from 1,4-diacetoxybutane, whereby the amounts of methanol and catalyst can be economized to a great extent as compared with the conventional process for producing tetrahydrofuran and 1,4-butanediol independently from 1,4-diacetoxybutane.

The above problem is solved according to the claims.

The invention provides a process for producing tetrahydrofuran and 1,4-butanediol simultaneously in any desired proportion from 1,4-diacetoxybutane which is characterized by (a) reacting 1,4-diacetoxybutane with an equimolar or a smaller quantity of water in the presence of an acidic catalyst, (b) separating the reaction mixture by distillation in a distillation column into a distillate comprising tetrahydrofuran as objective constituent and a bottom stream comprising the 1,4-diacetoxybutane as major constituent, (c) transesterification of at least a part of said bottom stream with methanol in the presence of an acidic or basic catalyst and (d) separating the reaction mixture by distillation in a reaction-distillation column into a bottom stream comprising 1,4-butanediol as major constituent and a distillate comprising methyl acetate as major constituent.

The steps (c) and (d) are preferably carried out simultaneously in a reaction-distillation column with counter-current gas-liquid contact.

The starting material used in this invention is 1,4-diacetoxybutane, which may contain up to 50 mole-% of mono-acetic esters of 1,4-butanediol such as 1-hydroxy-4-acetoxybutane and the like. 1,4-Diacetoxybutane can be obtained for example by hydrogenating 1,4-diacetoxybutene-2 synthesized by reacting butadiene, acetic acid and oxygen in the presence of a palladium catalyst. Though concerning the water, i.e. the other starting material for the cyclization reaction, there are no particular restrictions, it preferably contains no corrosive ions. The amount of water used in the reaction should be the

2

theoretical amount or less, i.e. the equimolar amount or less, based on the 1,4-diacetoxybutane supplied to the cyclization reaction. The water is preferably used in an amount of 0.2—0.8 mole per mole of 1,4-diacetoxybutane. It is one of the characteristic features of this invention to use the water in an equivalent amount or less. If the amount of water used exceeds the equimolar quantity, the conversion of water drops and the amount of water introduced into the subsequent step in the unreacted state increases, so that correspondingly more heat is required for its separation by distillation. If the amount of water is less than 0.2 mole, the one pass yield of tetrahydrofuran is too low and the amount of unreacted acetic ester to be recycled increases so that the reactor volume and the amount of the catalyst have to be increased, which is undesirable. Thus, an amount of water ranging from 0.2 to 0.8 mole is desirable in that the conversion of water is high and the amount of unreacted acetic ester recycled does not become too large.

Suitable acidic catalysts for the cyclization reaction are for example liquid acids such as sulfuric acid, phosphoric acid and the like and solid acids such as acidic cation exchange resins, solid phosphoric acid, silica-alumina and the like, among which the acidic cation exchange resins can be used with advantage in general.

The shape and material of the cyclization reactor may be varied depending on the kind of catalyst used. When an acidic cation exchange resin is used, a tubular packed reactor made of a material such as stainless steel of SUS 304 or SUS 316 grade or the like is used. When an acidic cation exchange resin is used, the reaction temperature is preferably 140°C or less if the heat resistance of the resin is considered. However, too low a temperature results in a reduced reaction efficiency, so that temperatures of 80°—130°C are preferable. The residence time is defined by the LHSV value (liquid space velocity) and may be varied depending on the reaction temperature. The LHSV value is selected in the range of 0.1—2.0 h$^{-1}$ and preferably in the range of 0.2—1.0 h$^{-1}$.

The distillation column used in step (b) may be usually employed distillation column, a multi-plate distillation column or a packed distillation column. Its material is preferably a stainless steel of SUS 316 grade in view of the acetic acid formed by the cyclization reaction (step (a)). Usually, the number of theoretical plates is about 5—30, the operation pressure is 6.7—10$^{-2}$—1.01 bar (50—760 Torr) and preferably 0.13—0.67 bar (100—500 Torr), and the reflux ratio is 0.1—3.

The distillate from the top of the column is a liquid which contains tetrahydrofuran as objective constituent and also contains water and acetic acid. This distillate is further purified by distillation and separated into a fraction comprising tetrahydrofuran as major constituent and a fraction of acetic acid. The number of distillations and the extent of rectification may be freely selected in accordance with the desired purity of the tetrahydrofuran.

The bottom stream of the distillation column comprises unreacted 1,4-diacetoxybutane as major constituent, which is sent to the subsequent reaction-distillation column, where it is subjected to transesterification. At this time, the bottom stream comprises not only the unreacted acetic ester but also 1-hydroxy-4-acetoxybutane formed by partial hydrolysis and 1,4-butanediol formed by complete hydrolysis of 1,4-diacetoxybutane, so that the amount of methanol used can be made smaller than that in the direct transesterification of 1,4-diacetoxybutane. Though the decrease of the methanol amount may vary depending on the contents of 1-hydroxy-4-acetoxybutane and 1,4-butanediol, 10 to 30% of the methanol can be saved usually. The methanol used in the transesterification may not necessarily be of high purity, but may contain methyl acetate formed as a by-product of the transesterification; the allowable content of methyl acetate in the methanol is 80 mole-% or less and preferably 60 mole-% or less. The amount of methanol used is 1.0—5.0 moles and particularly preferably 1.1—3.0 moles per mole of acetate groups of 1,4-diacetoxybutane. When methyl acetate containing methanol is used the mixture is used in such a manner that the amount of methanol in the mixture corresponds to the above-mentioned proportions. If the amount of methanol is less than 1.0 mole per mole of acetate groups, the transesterification does not proceed quantitatively. The use of more than 5.0 moles of methanol per mole of acetate groups gives no improvement and is disadvantageous because it unnecessarily increases the amount of liquid and the thermal load.

Suitable catalysts for the transesterification are for example liquid acids such as sulfuric acid, phosphoric acid and the like and solid acids such as acidic cation exchange resins, solid phosphoric acid, silica-alumina and the like as acidic catalysts and also sodium hydroxide, potassium hydroxide, sodium alkoxides, aluminum alkoxides, ammonia and the like as basic catalysts, among which sodium hydroxide and potassium hydroxide are inexpensive and usually most advantageously used. The amount of the catalyst used may be varied depending on its kind as well as on the proportions of 1-hydroxy-4-acetoxybutane and 1,4-butanediol contained in the bottom stream, so that it cannot be delt with in a general manner. However, when sodium hydroxide is used as the catalyst and a bottom stream containing 1-hydroxy-4-acetoxybutane and 1,4-butanediol is transesterified, the amount of sodium hydroxide can be made by 20—70% smaller as compared with using a bottom stream comprising 1,4-diacetoxybutane alone. Though a sodium hydroxide catalyst is advantageous from the industrial point of view, it can form, in some reactions, sodium acetate which is difficult to separate, so that it has been a problem to decrease the amount of catalyst. Now, according to this invention, the amount of catalyst can be decreased to a great extent. If the concentration of sodium hydroxide catalyst is too low, the reaction rate is low and the reaction stops before completion. If the concentration is too high, a useless salt is formed in a large quantity and its separation is difficult as mentioned above, which is undesirable. Therefore, it is usually preferable to use the sodium hydroxide catalyst in an amount of 0.1—10 moles and more preferably 0.2—5 moles per mole of 1,4-diacetoxybutane.

The reaction-distillation column for the transesterification may be of the same shape as generally used distillation columns, and a multi-plate distillation columns or packed distillation columns may be used. Though its material depends on the kind of catalyst, a steel of SS 41 grade is sufficient when a basic catalyst is used. In cases where an acidic catalyst is used, stainless steel of SUS 304 grade or SUS 316 grade and sometimes glass-lined steel may be used. The number of theoretical plates is about 5—30. The operation pressure is not particularly restricted and may be normal pressure, though the operation can also be conducted under pressure, if necessary. The reflux ratio in the operation is usually in the range of 0—2. The reaction temperature is dependent on the temperature at the bottom of the column. If the bottom temperature of the column is too high, there are formed by-products such as cyclic ethers, which is undesirable. If it is to low, the vapor of methanol cannot ascend, which is also undesirable. Therefore, the bottom temperature should be in the range of 70°—180°C and more preferably in the range of 75°—150°C.

The distillate from the top of the reaction-distillation column comprises methyl acetate as major constituent. When a basic catalyst is used, the bottom stream consists of 1,4-butanediol containing an alkali metal acetate.

When only a part of the bottom stream of the distillation column is supplied into the reaction-distillation column to carry out the transesterification with methanol, the residual amount of the bottom stream is recycled into the cyclization reactor as starting material for the production of tetrahydrofuran. In this case, the bottom stream of the distillation column is appropriately divided into the feed to the reaction-distillation column and the recycle into the cyclization reactor in accordance with the most advantageous proportion in the simultaneous production of tetrahydrofuran and 1,4-butanediol. For example, when water is used in an amount of 0.5 mole per mole of 1,4-diacetoxybutane, the one pass yield of tetrahydrofuran is about 35 mol-%. If the whole quantity of the unreacted 1,4-diacetoxybutane is directly subjected to transesterification, 1,4-butanediol is obtained in a yield of about 65 mole-%. Where tetrahydrofuran is to be produced in a proportion of more than 35 mole-%, the amount of water used is increased in the range of up to 0.8 mole or the unreacted acetic ester is recycled in an amount corresponding to the desired yield. Where tetrahydrofuran is to be produced in a proportion smaller than 35 mole-%, the amount of water used is made smaller than 0.5 mole. By such a procedure, tetrahydrofuran and 1,4-butanediol can be produced simultaneously in a substantially desired proportion.

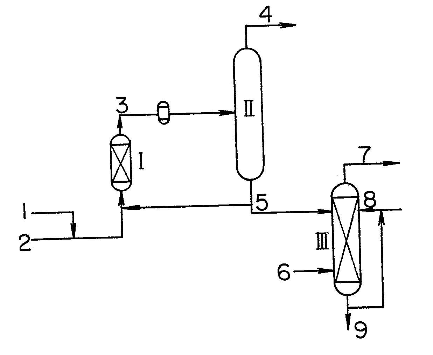

This invention is explained with reference to the accompanying drawing, which is a flow sheet illustrating an embodiment of this invention, wherein are:

| | |
|---|---|
| I | the cyclization reactor, |
| II | the distillation column, |
| III | the reaction-distillation column, |
| 1 | the feed line for water, |
| 2 | the feed line for 1,4-diacetoxybutane, |
| 3 | the discharge line for the cyclization mixture, |
| 4 | the discharge line for the tetrahydrofuran mixture, |
| 5 | the outlet line for the unreacted 1,4-diacetoxybutane, |
| 6 | the feed line for methanol, |
| 7 | the distillate line for methyl acetate mixture, |
| 8 | the feed line for the catalyst, and |
| 9 | the outlet line for the 1,4-butanediol mixture. |

The 1,4-diacetoxybutane is fed through line 2 and an equivalent or smaller quantity, based on the 1,4-diacetoxybutane, of water is fed through line 1, both to the reactor I. They are reacted in the presence of an acidic catalyst; the resulting reaction mixture is fed to the distillation column II through line 3. At this time, the reaction mixture may be neutralized by passing it through a bed of an anion exchange resin, if necessary. The distillate comprising tetrahydrofuran as objective constituent from the distillation column II contains some water and acetic acid, from which tetrahydrofuran can be obtained with high purity by appropriately combining subsequent distillations. On the other hand, the bottom stream of the distillation column II comprising unreacted 1,4-diacetoxybutane as major constituent is fed through line 5 to the upper part of the reaction-distillation column III. By recycling a part of the bottom stream, the production ratio of tetrahydrofuran to 1,4-butanediol can be controlled freely. In the reaction-distillation column III, the bottom stream is subjected to a counter-current contact reaction with methanol which is fed from the lower part of the column through line 6. As the methanol source, methanol containing methyl acetate may be used which can be obtained by partially hyrolyzing the methyl acetate by-product distilled out through line 7. The method of feeding the catalyst may be varied depending on the kind of the catalyst. When sodium hydroxide is used as the catalyst, it is dissolved in a part of the reaction mixture containing the diol or dissolved in methanol, and fed through

4

line 8 to the upper part of the reaction-distillation column III. After the reaction, a methanolic solution comprising 1,4-butanediol as major constituent but substantially free from unreacted acetic ester is obtained through line 9 as a bottom stream. From this mixture, 1,4-butanediol of high purity can be obtained by a simple distillation.

As mentioned above, in producing simultaneously tetrahydrofuran and 1,4-butanediol from 1,4-diacetoxybutane, the process of this invention makes it possible to reduce the amount of water used to a great extent as compared with the case of direct hydrolysis of 1,4-diacetoxybutane. Furthermore, the amounts of methanol and catalyst used can also be reduced, because 1-hydroxy-4-acetoxybutane formed by partial hydrolysis and 1,4-butanediol formed by total hydrolysis are used in the transesterification, and the two products can be produced in any desired proportion. Therefore, the process of this invention is quite advantageous in industry.

This invention is illustrated more specifically below with reference to Examples and a Comparative Example. The Example is only illustrative.

Example 1

A reactor made of a stainless steel of SUS 316 grade, having an inner diameter of 50 mm and a height of 700 mm and equipped with a heating jacket was used as cyclization reactor, the inner space of which was packed with 1.3 l of a commercially available acidic cation exchange resin (Amberlite 200), converted into the sulfonic acid form by treatment with hydrochloric acid, as a catalyst bed. As the distillation column, a distillation column made of a stainless steel of SUS 316 grade, having an inner diameter of 25 mm and a height of 2.0 m and equipped with a heating jacket was used, the inner space of which was packed with McMahon packings made of a stainless steel of SUS 316 grade and which was provided at the bottom with a 500-ml still equipped with a heating jacket. As the reaction-distillation column, a distillation column made of a stainless steel of SUS 316 grade, having an inner diameter of 25 mm and a height of 1.5 m and equipped with a heating jacket was used, the inner space of which was packed with McMahon packings made of a stainless steel of SUS 316 grade and which was provided at the bottom with a 500-ml still equipped with a heating jacket. After connecting the above-mentioned means through pumps, etc., a continuous reaction was carried out according to the accompanying drawing.

Water was fed through line 1 at a rate of 27 g/h and 1,4-diacetoxybutane was fed through line 2 at a rate of 522 g/h, both continuously and after being preheated to 120°C, to the reactor I. The reaction mixture was taken out through line 3, passed through a tank packed with 130 ml of commercial anion exchange resin (Amberlite IRA 400) treated with caustic soda, and then fed to the distillation column II. The distillation column II was continuously operated at a pressure of 0.27 bar (200 Torr), at a column bottom temperature of 200°C and at a reflux ratio of 0.5. The distillate was continously taken out from line 4 at a rate of 217 g/h, from which tetrahydrofuran having a purity of 99.9% or more was obtained at a rate of 75 g/h by another combination of distillations.

On the other hand, the bottom stream (332 g/h), withdrawn from the bottom of the distillation column II passed line 5 and was cooled to 100°C, after which it was fed to a position 100 mm below the top of the reaction-distillation column III. Methanol was fed to a position 400 mm above the bottom of the reaction-distillation column at a rate of 219 g/h. Sodium hydroxide as the catalyst was dissolved in the recycled part of the reaction mixture to give a 2% solution and this solution was fed through line 8 to a position 150 mm below the top at a rate of 90 g/h. While keeping the inner temperature of the still at 120°C and continuously operating the apparatus under normal pressure at a reflux ratio of 0, the distillate was taken out through line 7 at a rate of 361 g/h; the bottom stream was taken out through line 9 at a rate of 192 g/h. From the bottom stream, 1,4-butanediol having a purity of 99% or more was obtained by a simple distillation at a rate of 171 g/h. The molar ratio of tetrahydrofuran to 1,4-butanediol thus obtained was 35:65.

In this experiment, the chemical compositions at the principal locations were as follows, wherein the locations are expressed in accordance with the reference numerals used in the drawing:

| 4 | Tetrahydrofuran | 29.2 mole-% |
|---|---|---|
|   | Water | 7.5 mole-% |
|   | Acetic acid | 63.3 mole-% |
| 5 | 1,4-Diacetoxybutane | 90.8 mole-% |
|   | 1-Hydroxy-4-acetoxybutane | 9.2 mole-% |
|   | 1,4-Butanediol | Trace |

**0 012 376**

| | | |
|---|---|---|
| 7 | Methyl acetate | 57.5 mole-% |
| | Methanol | 42.5 mole-% |
| 9 | Methanol | 16.7 mole-% |
| | 1-Hydroxy-4-acetoxybutane | 0.8 mole-% |
| | 1,4-Butanediol | 82.5 mole-% |

### Comparative Example

The transesterification of Example 1 was repeated, except that pure 1,4-diacetoxybutane was substituted for the bottom stream withdrawn from the distillation column II.

The 1,4-diacetoxybutane was fed to a position 100 mm below the top of the reaction-distillation column III in the same molar (339 g/h) as the bottom stream of the first distillation column. Methanol was fed to a position 400 mm above the bottom of the reaction-distillation column III at the same rate as in Example 1 (219 g/h). Sodium hydroxide as the catalyst was dissolved in the recycled part of the reaction mixture to form a 4% solution (twice the concentration of Example 1) and this solution was fed to a position 150 mm below the top at a rate of 90 g/h.

While operating the apparatus in the same manner as in Example 1, the distillate was taken out through line 7 at a rate of 365 g/h, while the bottom stream was taken out through line 9 at a rate of 197 g/h. From the bottom stream, 1,4-butanediol having a purity of 94.8 mole-% was obtained at a rate of 174 g/h by the same distillation as in Example 1. The remainder was unreacted 1-hydroxy-4-acetoxybutane. That is to say, due to the insufficient supply of methanol, there was obtained only 1,4-butanediol having a low purity in spite of the increased amount of catalyst.

In the same manner as in Example 1, the chemical compositions at the principal locations are shown below in accordance with the reference numerals used in the drawing.

| | | |
|---|---|---|
| 7 | Methyl acetate | 58.7 mole-% |
| | Methanol | 41.3 mole-% |
| 9 | Methyl acetate | 0.9 mole-% |
| | Methanol | 15.8 mole-% |
| | 1-Hydroxy-4-acetoxybutane | 4.3 mole-% |
| | 1,4-Butanediol | 79.1 mole-% |

### Example 2

The same procedure as in Example 1 was repeated in the same apparatus as in Example 1, except that a part of the bottom stream from the distillation column was recycled to the reactor to subject it to cyclization; simultaneously methyl acetate containing methanol was used as the starting methanol for the transesterification. The apparatus has been operated continuously.

To the reactor were fed water at a rate of 36 g/h through line 1, 1,4-diacetoxybutane at a rate of 522 g/h through line 2 and a part of the bottom stream from the distillation column II at a rate of 179 g/h, said bottom stream having been taken out through line 5 and containing 1-hydroxy-4-acetoxybutane. The reaction was effected under the same conditions as in Example 1.

The reaction mixture was taken out through line 3 and fed to the distillation column II, from which a distillate was taken out at a rate of 289 g/h through line 4. This distillate was purified by distillation to obtain tetrahydrofuran with a purity of 99.9% or more at a rate of 101 g/h.

On the other hand, the remainder of the bottom stream from the distillation column II was fed to a position 100 mm below the top of the reaction-distillation column III at a rate of 269 g/h and methanol containing 20 mole-% of methyl acetate was fed to a position 400 mm above the bottom of the reaction-distillation column III at a rate of 319 g/h. Sodium hydroxide as the catalyst was dissolved in a part of the reaction mixture taken out from the bottom of the column to form a 2% solution; this solution was fed to a position 150 mm below the top of the reaction-distillation column III at a rate of 73 g/h as in Example 1.

The apparatus was operated continuously as in Example 1 while taking out a distillate at a rate of 435 g/h through line 7 and a bottom stream at a rate of 153 g/h through line 9. The bottom stream was purified by simple distillation to obtain 1,4-butanediol with a purity of 99% or more at a rate of 139 g/h.

The molar ratio of the tetrahydrofuran obtained to the 1,4-butanediol obtained was 48:52. The chemical compositions at the principal locations in this experiment were as follows, wherein the locations are expressed in accordance with the reference numerals used in the drawing:

6

| 4 | Tetrahydrofuran | 29.5 mole-% |
|---|---|---|
|   | Water | 8.6 mole-% |
|   | Acetic acid | 61.9 mole-% |
| 5 | 1,4-Diacetoxybutane | 90.8 mole-% |
|   | 1-Hydroxy-4-acetoxybutane | 9.2 mole-% |
|   | 1,4-Butanediol | trace |
| 7 | Methyl acetate | 60.4 mole-% |
|   | Methanol | 39.6 mole-% |
| 9 | Methanol | 16.7 mole-% |
|   | 1-Hydroxy-4-acetoxybutane | 0.8 mole-% |
|   | 1,4-Butanediol | 82.5 mole-% |

## Claims

1. A process for producing simultaneously tetrahydrofuran and 1,4-butanediol in any desired proportion, characterized by
   (a) reacting 1,4-diacetoxybutane with an equimolar or a smaller quantity of water in the presence of an acidic catalyst,
   (b) separating the obtained reaction mixture by distillation into a distillate comprising tetrahydrofuran as objective constituent and a bottom stream comprising the 1,4-diacetoxybutane as main constituent and
   (c) transesterification of at least a part of said bottom stream with methanol in the presence of an acidic or basic catalyst and
   (d) separating the reaction mixture by distillation into a bottom stream comprising 1,4-butanediol as main constituent and a distillate comprising methyl acetate as main constituent.

2. A process according to claim 1, characterized in that the steps (c) and (d) are carried out simultaneously in a reaction-distillation column with counter-current gas-liquid contact.

3. A process according to claim 1 or 2, characterized in that in step (a) 0.2—0.8 mole of water per mole of 1,4-diacetoxybutane are used.

4. A process according to one of claims 1—3, characterized in that in step (c) a mixture of 20 mole-% or more of methanol and 80 mole-% or less of methyl acetate is used.

5. A process according to claim 4, characterized in that in step (c) a mixture of 40 mole-% or more of methanol and 60 mole-% or less of methyl acetate is used.

6. A process according to one of claims 1—5, characterized in that 1—5 moles of methanol per mole of ester groups of the 1,4-diacetoxybutane in the bottom stream are used.

7. A process according to one of claims 1—6, characterized in that in step (a) sulfuric acid, phosphoric acid, an acidic cation exchange resin, solid phosphoric acid or silica-alumina are used as acidic catalyst.

8. A process according to one of claims 1—7, characterized in that in step (c) sulfuric acid, phosphoric acid, an acidic cation exchange resin or silica-alumina are used as acidic catalyst.

9. A process according to one of claims 1—8, characterized in that in step (c) sodium hydroxide, potassium hydroxide, a sodium alkoxide, an aluminium alkoxide or ammonia are used as basic catalyst.

## Revendications

1. Procédé pour la préparation simultanée du tetrahydrofuranne et du 1,4-butanediol dans des proportions quelconques, caractérisé par
   (a) réaction de 1,4-diacétoxybutane avec une quantité équimolaire ou inférieure d'eau en présence d'un catalyseur acide,
   (b) séparation du mélange réactionnel obtenu par distillation en un distillat contenant le tetrahydrofuranne comme constituant désiré et un produit de fond contenant le 1,4-diacétoxybutane comme constituant principal,
   (c) transestérification d'au moins une partie du produit de fond avec du méthanol en présence d'un catalyseur acide ou basique et
   (d) séparation du mélange réactionnel par distillation en un produit de fond contenant le 1,4-

**0 012 376**

butanediol comme constituant principal et un distillat contenant l'acétate de méthyle comme constituant principal.

2. Procédé selon la revendication 1, caractérisé en ce que les étapes (c) et (d) sont effectuées simultanément dans une colonne de réaction-distillation avec contact gazliquide en contre-courant.

3. Procédé selon la revendication 1 ou 2, caractérisé en ce qu'on utilise dans l'étape (a) 0,2—0,8 moles d'eau par mole 1,4-diacétoxybutane.

4. Procédé selon l'une quelconque des revendications 1—3, caractérisé en ce qu'on utilise dans l'étape (c) un mélange d'au moins 20 mole-% de méthanol et d'au plus 80 mol-% de l'acétate de méthyle.

5. Procédé selon la revendication 4, caractérisé en ce qu'on utilise dans l'étape (c) un mélange d'au moins 40 mole-% de méthanol et d'au plus 60 mole-% de l'acétate de méthyle.

6. Procédé selon l'une quelconque des revendications 1—5, caractérisé en ce qu'on utilise 1—5 moles de méthanol par mole de groupes ester du 1,4-diacétoxybutane dans le produit de fond.

7. Procédé selon l'une quelconque des revendications 1—6, caractérisé en ce qu'on utilise dans l'étape (a) de l'acide sulfurique, de l'acide phosphorique, une résine échangeuse de cations acide, de l'acide phosphorique solide ou silice-alumine comme catalyseur acide.

8. Procédé selon l'une quelconque des revendications 1—7, caractérisé en ce qu'on utilise dans l'étape (c) de l'acide sulfurique, de l'acide phosphorique, une résine échangeuse de cations acide ou silice-alumine comme catalyseur acide.

9. Procédé selon l'une quelconque des revendications 1—8, caractérisé en ce qu'on utilise dans l'étape (c) de la soude, de la potasse, un alkoxyde de sodium, un alkoxyde d'aluminium ou de l'ammoniaque comme catalyseur basique.

**Patentansprüche**

1. Verfahren zur gleichzeitigen Herstellung von Tetrahydrofuran und 1.4-Butandiol in beliebigem Verhältnis, gekennzeichnet durch
   (a) Umsetzung von 1.4-Diacetoxybutan mit einer äquimolaren oder geringeren Menge Wasser in Gegenwart eines sauren Katalysators,
   (b) Auftrennung des erhaltenen Reaktionsgemischs durch Destillation in ein Tetrahydrofuran als angestrebte Verbindung enthaltendes Destillat und einen Ablauf, der 1.4-Diacetoxybutan als Hauptbestandteil enthält,
   (c) Umesterung zumindest eines Teils des Ablaufs mit Methanol in Gegenwart eines sauren oder basischen Katalysators und
   (d) Auftrennung des Reaktionsgemischs durch Destillation in einen Ablauf mit 1.4-Butandiol als Hauptbestandteil und ein Destillat mit Methylacetat als Hauptbestandteil.

2. Verfahren nach Anspruch 1, dadurch gekennzeichnet, daß die Schritte (c) und (d) zugleich in einer Reaktions-Destillationskolonne mit Gas-Flüssig-Kontakt im Gegenstrom durchgeführt werden.

3. Verfahren nach Anspruch 1 oder 2, dadurch gekennzeichnet, daß in Schritt (a) 0,2—0,8 mol Wasser pro mol 1.4-Diacetoxybutan eingesetzt werden.

4. Verfahren nach einem der Ansprüche 1 bis 3, dadurch gekennzeichnet, daß in Schritt (c) ein Gemisch von mindestens 20 mol-% Methanol und höchstens 80 mol-% Methylacetat eingesetzt wird.

5. Verfahren nach Anspruch 4, dadurch gekennzeichnet, daß in Schritt (c) ein Gemisch von mindestens 40 mol-% Methanol und höchstens 60 mol-% Methylacetat eingesetzt wird.

6. Verfahren nach einem der Ansprüche 1 bis 5, dadurch gekennzeichnet, daß 1 bis 5 mol Methanol pro mol Estergruppen des 1.4-Diacetoxybutans im Ablauf eingesetzt werden.

7. Verfahren nach einem der Ansprüche 1 bis 6, dadurch gekennzeichnet, daß in Schritt (a) Schwefelsäure, Phosphorsäure, ein saurer Kationenaustauscher, feste Phosphorsäure oder Siliciumoxid-Aluminiumoxid als saurer Katalysator eingesetzt wird.

8. Verfahren nach einem der Ansprüche 1 bis 7, dadurch gekennzeichnet, daß in Schritt (c) Schwefelsäure, Phosphorsäure, ein saurer Kationenaustauscher oder Siliciumoxid-Aluminiumoxid als saurer Katalysator eingesetzt wird.

9. Verfahren nach einem der Ansprüche 1 bis 8, dadurch gekennzeichnet, daß in Schritt (c) Natriumhydroxid, Kaliumhydroxid, ein Natriumalkoxid, ein Aluminiumalkoxid oder Ammoniak als basischer Katalysator eingesetzt wird.